# DEMANDE DE BREVET EUROPEEN

(11) **EP 0 940 140 A1**
(43) Date de publication de la demande: **08.09.1999**
(21) Numéro de dépôt: 98403335.7
(22) Date de dépôt: 30.12.1998
(51) Int. Cl.: A61K 31/19

(54) **Composition à base d'alpha-hydroxy-acides et de céramides utilisable par voie topique pour le traitement d'affections dermatologiques**

(30) Priorité: 30.12.1997 FR 9716674
(71) Demandeur: Laboratoire D'Evolution Dermatologique (L.E.D.), 75008 Paris (FR)
(72) Inventeur: Estanove, Cyril, 92100 Boulogne (FR)
(74) Mandataire: L'Helgoualch, Jean

(57) **Abrégé**

L'invention concerne une composition à base d'alpha-hydroxy-acides et de céramides utilisable en dermatologie.

La composition comprend en combinaison au moins un alpha-hydroxy-acide, ou un de ses sels et esters acceptables, et au moins un céramide ou précurseur de céramide. De préférence, elle comprend un alpha-hydroxy-acide, un sel d'ammonium d'alpha-hydroxy-acide, et un céramide ou un précurseur de céramide choisi parmi la sphingosine ou la phytosphingosine.

Application au traitement d'affections dermatologiques par voie topique.

## Description

La présente invention concerne une composition pharmaceutique et cosmétique, et plus particulièrement une composition comprenant en combinaison au moins un alpha-hydroxy-acide et au moins un céramide ou un précurseur de céramide tel que la sphingosine ou la phytosphingosine, utilisable par administration topique pour les traitements et soins de la peau.

L'utilisation de divers alpha-hydroxy-acides, y compris leurs sels et esters, est bien connue dans des compositions utiles en thérapeutique ou en cosmétique. Ainsi, le brevet EP 273.202 décrit l'utilisation d'alpha-hydroxy-acides comme additifs dans des compositions contenant un ou plusieurs principes actifs de médicament, afin d'en améliorer l'activité par voie topique. Le brevet EP 504.862 montre que certains alpha-hydroxy-acides peuvent être utilisés pour le traitement de l'atrophie de la peau induite par l'application topique de corticoïdes tels que le propionate de clobetasol.

Le brevet EP 413.528 décrit des compositions utilisables pour le traitement de diverses affections dermatologiques telles que l'acné, l'eczéma ou le psoriasis, contenant des alpha-hydroxy-acides en combinaison avec un agent amphotère ayant pour effet d'augmenter le pH de la composition.

On connaît aussi des produits disponibles dans le commerce comme celui connu sous la marque LacHydrin® comprenant une solution à 12% d'acide lactique neutralisé par de l'hydroxyde d'ammonium, de pH voisin de 5 (Physician Desk reference, p. 2285, 1990), utilisable pour le traitement de certaines dermatoses telles que xérose ou ichtyose.

Ainsi, les propriétés connues des alpha-hydroxy-acides permettent d'envisager leur utilisation en dermatologie pour le traitement de certaines affections de la peau. Cependant, ils doivent être utilisés à des concentrations relativement faibles, n'excédant pas 10%, en raison des effets secondaires qu'ils peuvent entraîner, par exemple une plus grande sensibilité et une irritation de la peau.

D'autre part, les céramides sont des constituants naturels des couches superficielles de la peau, et notamment le stratum corneum, et jouent un rôle essentiel dans la structure de la barrière lipidique cutanée, mais leur teneur tend à diminuer avec l'âge dans des proportions importantes. C'est pourquoi de nombreuses recherches ont été faites pour pouvoir disposer de céramides synthétiques susceptibles d'être utilisés dans des compositions utilisables en cosmétologie, en particulier pour lutter contre le dessèchement de la peau, ou dans des compositions pour traitement capillaire. Par exemple, les demandes de brevet FR-A-2.673.179 et FR-A-2.714.829 décrivent des céramides utilisables dans des compositions cosmétiques ou dermatologiques.

On sait aussi que la phytosphingosine et la sphingosine, précurseurs de céramides, sont présents dans la peau humaine, et des études ont montré que ces molécules ont des propriétés inhibitrices de la protéine kinase C, et semblent impliquées dans la différenciation des kératinocytes de l'épiderme. On a également observé que des sphingosines présentes dans le stratum corneum et d'autres couches de l'épiderme, inhibent la croissance de certains micro-organismes indésirables.

Selon la demande de brevet WO-95.03028, la sphingosine, utilisée dans une composition à pH voisin de 5, pourrait réduire l'effet irritant de certains alpha-hydroxy-acides, tandis que les céramides n'auraient aucun effet.

Le brevet US-A-5.690.947 décrit une composition cosmétique à base d'alpha-hydroxy-acides contenant de l'huile de bourrache à effet anti-irritant.

Les travaux entrepris par la demanderesse ont permis de montrer que d'excellents résultats peuvent être obtenus en combinant certains alpha-hydroxy-acides avec certains céramides ou précurseurs de céramides, tels que la sphingosine et la phytosphingosine, au sein d'une même composition.

La présente invention a pour objet une composition à base d'alpha-hydroxy-acide et de céramide ou de précurseur de céramide, tel que la sphingosine et la phytosphingosine, utilisable en thérapeutique et en cosmétique par application topique.

L'invention a encore pour objet une composition comprenant au moins un alpha-hydroxy-acide ou un de ses sels ou esters acceptables en dermatologie, en combinaison avec un céramide ou un précurseur de céramide tel que la sphingosine et la phytosphingosine, ainsi que des excipients adaptés à l'application par voie topique, pour le traitement de diverses affections dermatologiques.

Suivant une première forme de réalisation, la composition suivant la présente invention comprend, en combinaison, au moins un alpha-hydroxy-acide ou un de ses sels ou esters, et au moins un céramide ou un précurseur de céramide, tel que la sphingosine et la phytosphingosine.

Suivant une autre forme de réalisation, la composition suivant la présente invention comprend, en combinaison, au moins un alpha-hydroxy-acide ou un de ses sels ou esters, un sel d'ammonium d'alpha-hydroxy-acide, et au moins un céramide ou un précurseur de céramide, tel que la sphingosine et la phytosphingosine.

Les alpha-hydroxy-acides utilisables dans les compositions suivant la présente invention, ainsi que leurs sels et esters, peuvent être représentés par la formule générale (I) suivante : 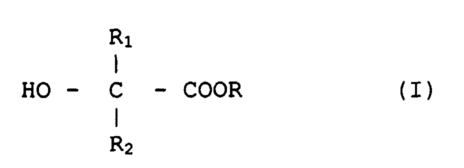 dans laquelle R₁ et R₂, identiques ou différents, représentent un atome d'hydrogène, un groupe alkyle linéaire ou ramifié comprenant 1 à 5 atomes de carbone éventuelement substitué par un groupe carboxyle, ou un groupe phényle, et R est un atome d'hydrogène, un groupe ammonium ou un métal alcalin ou alcalino-terreux.

Dans la formule (I) ci-dessus, R₁ est de préférence un groupe méthyle ou éthyle et R₂ est un atome d'hydrogène, ou R₁ et R₂ représentent tous deux un groupe -CH₂COOH. R est de préférence un atome d'hydrogène, un groupe ammonium ou un atome de sodium, de magnésium, de potassium ou de calcium. Quand R est un atome d'hydrogène, la formule (I) représente un alpha-hydroxy-acide qui peut être de préférence l'acide glycolique, l'acide lactique, l'acide mandélique, l'acide citrique ou l'acide 2-hydroxybutanoïque.

Les sels et esters représentés par la formule générale (I) où R est un groupe ammonium ou un atome de métal alcalin ou alcalino-terreux, peuvent être obtenus par une simple réaction acido-basique, en faisant réagir l'acide représenté par la même formule (I) où R est un atome d'hydrogène, avec une base constituée par exemple par un hydroxyde, tel que l'hydroxyde de magnésium ou de calcium, ou un hydroxyde de métal alcalin ou alcalino-terreux.

Suivant une forme préférentielle de réalisation de l'invention, on utilise un sel d'ammonium d'alpha-hydroxy-acide représenté par la formule générale (I) où R est un groupe ammonium, et plus préférentiellement le lactate d'ammonium, le citrate d'ammonium, le glycolate d'ammonium, ou le mandélate d'ammonium.

Ce sel d'ammonium d'alpha-hydroxy-acide est utilisé de préférence en combinaison avec un alpha-hydroxy-acide et un céramide ou un précurseur de céramide, ces trois composants de base étant complétés par des excipients et supports habituellement utilisés dans les compositions cosmétiques et pharmaceutiques. Par exemple, il peut être avantageux d'utiliser une composition comprenant un lactate ou un glycolate d'ammonium, un céramide, et un ou deux alpha-hydroxy-acides tels que l'acide glycolique, l'acide mandélique et l'acide malique. Les essais effectués ont montré que la présence combinée d'un sel d'ammonium et d'un ou plusieurs acides potentialise les effets du sel d'ammonium en améliorant l'absorption et la rémanence.

La composition suivant la présente invention contient encore avantageusement un β-glucan ou un dérivé de β-glucan permettant de lui conférer un meilleur effet anti-vieillis-sement de la peau et d'excellentes propriétés de renforcement de la résistance naturelle de la peau contre les effets des rayons ultraviolets.

Les β-glucans sont des polyglucoses comportant des liaisons β-glucosidiques entre l'oxygène en C₁ d'une molécule de glucose et l'un des quatre hydroxyles en C₂, C₃, C₄ et C₆ de la molécule suivante. Les dérivés carboxyméthylés présentent l'avantage d'être bien solubles dans l'eau pour un degré de carboxyméthylation variant de 50 % à 75 % environ. Les études effectuées sur ces β-glucans ont montré qu'ils sont capables de stimuler le système immunitaire humain, et s'avèrent efficaces pour réduire les effets nocifs des radiations ultraviolettes sur la peau.

Les céramides utilisés dans la présente invention peuvent résulter de la N-acylation de la sphingosine ou de la phytosphingosine. La sphingosine, ou 2-amino-4-octadécène-1,3-diol, est la base la plus importante des sphingolipides animaux. La phytosphingosine, ou 2-amino-1,3,4-octadécane-triol, se trouve à l'état naturel dans les lipides des plantes, où l'on trouve également certains analogues à 16, 19 et 20 atomes de carbone. Suivant la présente invention, les dénominations sphingosine et phytosphingosine utilisées ici englobent également ces analogues.

Comme indiqué ci-dessus, les céramides utilisables dans l'invention peuvent avoir pour base la chaîne sphingosine ou la chaîne phytosphingosine, selon le type de céramide. Ainsi les céramides de type 1, 2, 4 et 5 ont pour base la chaîne sphingosine, tandis que les céramides 3 et 6 ont pour base la chaîne phytosphingosine. Les céramides utilisés dans les compositions suivant la présente invention sont de préférence des céramides identiques aux céramides naturels de la peau, que l'on peut obtenir par des procédés biotechnologiques.

Suivant l'invention, il est possible d'utiliser un céramide et un précurseur, tel que la sphingosine ou la phytosphingosine, isolément ou en association, le choix étant fait en fonction des propriétés recherchées.

Les compositions suivant l'invention comprennent de préférence entre 0,1 % et 15 % en poids d'alpha-hydroxy-acide et entre 0,01 % et 10 % en poids de céramide ou de précurseur de céramide par rapport au poids total de la composition. Suivant une forme préférentielle de réalisation, les compositions comprennent un ou plusieurs alpha-hydroxy-acides à raison de 0,1 % à 10 %, un sel d'ammonium d'alpha-hydroxy-acide à raison de 0,5 % à 50 % et un céramide à raison de 0,01 % à 5 % en poids. Une composition préférée, suivant l'invention, comprend entre 0,1 % et 6 % en poids d'alpha-hydroxy-acide, entre 0,5 % et 18 % de sel d'ammonium d'alpha-hydroxy-acide, et entre 0,01 % et 1 % de céramide, par rapport au poids total de la composition. Une autre composition préférée de la composition de l'invention contient notamment entre 1 % et 18 % de sel d'ammonium d'alpha-hydroxy-acide, entre 0,01 % et 1 % de céramide, et entre 0,01 % et 1 % de β-glucan ou de β-glucan carboxyméthylé par rapport au poids total de la composition.

Les compositions conformes à la présente invention sont destinées à être administrées par voie topique et comprennent donc, outre les composants essentiels indiqués ci-dessus, des excipients, additifs et supports usuels acceptables sur le plan pharmaceutique et cosmétique. Ainsi, on peut utiliser des émulsifiants, des conservateurs, des épaississants, des colorants, des antioxydants, des agents hydratants, des parfums et divers additifs destinés à améliorer les propriétés de la composition. Il est également possible d'utiliser des systèmes connus d'encapsulation, par exemple au moyen de liposomes.

On peut encore incorporer dans la composition de l'invention des produits connus pour leur propriétés de filtrage ou de protection contre les rayons ultraviolets, et en particulier contre les rayons UV-B (de longueur d'onde comprise entre 290 et 320 nm) qui sont souvent responsables d'apparitions d'érythèmes ou de brûlures cutanées, et contre les rayons UV-A (longueur d'onde comprise entre 320 et 400 nm) qui sont responsables du brunissement mais peuvent aussi provoquer une altération et un vieillissement prématuré de la peau en cas d'expositions prolongées au soleil. Ces additifs peuvent être constitués par des particules formant écran ou des filtres, choisis en fonction du degré de protection recherché. On peut utiliser des filtres tels que des dérivés de triazine, de benzophénone, de camphre (par exemple un méthylbenzylidène-camphre), de diphényl-acrylate, ou de dibenzoylméthane (par exemple un t-butyl-méthoxy-dibenzoylméthane. On peut aussi utiliser des écrans à base pigments d'oxydes métalliques, et par exemple des particules d'oxyde de titane, de zinc ou de zirconium.

Les compositions peuvent se présenter sous forme de crèmes, laits, gels, lotions, masques ou pommades, et peuvent être utilisées en particulier pour le traitement d'affections dermatologiques telles que dermites atopiques, xéroses, psoriasis, acné, contre le vieillissement cutané et pour la protection de la peau contre les effets des rayons UV.

Les essais effectués avec les compositions suivant l'invention ont mis en évidence d'intéressantes propriétés utilisables plus particulièrement en cosmétologie, et notamment une excellente efficacité résultant d'une meilleure pénétration des sels d'ammonium dans l'épiderme, ainsi qu'une action restructurante exercée sur l'épiderme assurant un traitement efficace contre le vieillissement cutané, se traduisant notamment par un effet lissant et restructurant de la peau et une atténuation des rides et ridules. Des essais comparatifs effectués avec une composition conforme à l'invention, d'une part, et une composition similaire mais ne contenant pas de céramide, d'autre part, montrent que des résultats nettement meilleurs sont obtenus avec la composition de l'invention.

Les exemples suivants illustrent l'invention plus en détail sans en limiter la portée. Sauf indication contraire, toutes les parties et pourcentages sont exprimés en poids.

### Exemple 1

On prépare une crème ayant la composition pondérale suivante :

| | |
|---|---|
| Lactate d'ammonium (sol. aqueuse à 65%) | 12,00 |
| Acide malique | 0,80 |
| Acide glycolique | 0,70 |
| Céramide 6 | 0,15 |
| Glycérine | 5,00 |
| Alcool céto-stéarylique | 7,00 |
| Dimethicone | 1,00 |
| Isohexadécane | 4,00 |
| Malate de di-(C12-C13))-alkyle | 4,00 |
| Octénylsuccinate d'aluminium | 4,00 |
| Parfum | 0,30 |
| Eau déminéralisée, q.s.p. | 100,00 |

La crème dont la composition est donnée ci-dessus est destinée à être appliquée sur le visage une ou deux fois par jour.

Elle peut être préparée par les techniques classiques. Par exemple, on peut obtenir une crème ayant la composition ci-dessus en opérant suivant la méthode indiquée ci-après, sans que celle-ci soit limitative : on mélange dans un fondoir chauffé à environ 85°C les composants de la phase grasse, c'est-à-dire essentiellement la glycérine et l'alcool céto-stéarylique, et le céramide 6. D'autre part, on prépare dans une cuve une solution aqueuse contenant environ 90% de l'eau nécessaire à la composition, le lactate d'ammonium ainsi que les alpha-hydroxy-acides, et on mélange soigneusement en chauffant à 75°C environ.

On prépare ensuite l'émulsion en versant la phase aqueuse dans la phase grasse sous agitation continue, et en laissant revenir à une température voisine de la température ambiante, puis on ajoute les parfums ainsi que l'eau restante, et on maintient sous agitation pendant environ 20 min.

### Exemple 2

Par une méthode classique et au moyen d'un appareillage usuel, on prépare un lait ayant la composition suivante :

| | |
|---|---|
| Lactate d'ammonium (sol. aqueuse à 65%) | 15,40 |
| Acide malique | 1,00 |
| Acide glycolique | 0,90 |
| Céramide 3b | 0,10 |
| Huile d'amandes douces | 1,00 |
| Lactate de cétyle | 0,50 |
| Alcool cétylique | 0,50 |
| Acide oléique | 0,60 |
| Benzoate de C12-C15 | 2,00 |
| Parfum | 0,20 |
| Eau, q.s.p. | 100,00 |

Ce lait est utilisé en application sur le corps une ou deux fois par jour.

### Exemple 3

On prépare une crème ayant la composition pondérale indiquée ci-après :

| | |
|---|---|
| Acide mandélique | 5,00 |
| Phytosphingosine | 0,20 |
| Lactate d'ammonium | 5,00 |
| Polyacrylamide (and) isoparaffine C13-14 (and) laurate d'éthyle | 2,50 |
| Succinate de dioctyle | 2,00 |
| Cyclométhicone | 4,00 |
| PPG-3 myristyl éther | 2,00 |
| PEG-7 glycéryl cocoate | 2,00 |
| Propylène glycol | 2,00 |
| Nylon-12 | 3,00 |
| Parfum | 0,30 |
| Eau q.s.p. | 100,00 |

### Exemple 4

On prépare une crème ayant la composition suivante :

| | |
|---|---|
| Acide linoléique | 3,00 |
| Acide mandélique | 5,00 |
| Phytosphingosine | 0,20 |
| Lactate d'ammonium | 5,00 |
| Polyglucoside de siloxane | 10,00 |
| Méthacrylate de polyméthyle | 2,50 |
| Cétyl diméthicone copolyol | 1,50 |
| Cyclométhicone | 5,00 |
| parfum | 0,30 |
| Eau q.s.p. | 100,00 |

### Exemple 5

On prépare une crème ayant la composition suivante :

| | |
|---|---|
| Acide mandélique | 5,00 |
| Phytosphingosine | 0,20 |
| Polyacrylamide (and) isoparaffine (and) laurate d'éthyle | 3,50 |
| Succinate de dioctyle | 2,00 |
| PEG-100 stéarate (and) stéarate de glycéryle | 2,00 |
| PPG-3 myristyl éther | 2,00 |
| PEG-7 glycéryl cocoate | 2,00 |
| Cyclométhicone | 4,00 |
| Propylène glycol | 2,00 |
| Nylon-12 | 3,00 |
| Parfum | 0,30 |
| Eau q.s.p. | 100,00 |
| Hydroxyde de sodium, q.s.p. pH 3,5 | |

### Exemple 6

On prépare une crème ayant la composition suivante :

| | |
|---|---|
| Acide mandélique | 5,00 |
| Phytosphingosine | 0,30 |
| Mandélate d'ammonium | 3,00 |
| Arachidyl béhényl arachidyl glucoside | 2,00 |
| Succinate de dioctyle | 2,00 |
| PEG-100 stéarate (and) stéarate de glycéryle | 2,00 |
| PPG-3 myristyl éther | 2,00 |
| Cyclométhicone | 3,00 |
| Acide linoléique | 3,00 |
| Nylon-12 (and) lauroyl lysine | 2,50 |
| Parfum | 0,30 |
| Eau q.s.p. | 100,00 |
| Hydroxyde de sodium, q.s.p. pH 3,5 | |

### Exemple 7

On prépare une crème plus particulièrement destinée à la protection de la peau, ayant la composition suivante :

| | |
|---|---|
| Lactate d'ammonium (sol. aqueuse à 65%) | 12,30 |
| Acide citrique | 1,20 |
| Céramide 6 | 0,15 |
| β-glucan carboxyméthylé (sel de sodium) | 0,15 |
| Glycérine | 5,00 |
| Dimethicone | 1,50 |
| Triglycérides C8/C10 | 6,00 |
| Acide stéarique | 0,50 |
| Stéareth-2 | 2,00 |
| Stéareth-21 | 2,50 |
| Isohexadécane | 6,00 |
| PEG-100 stéarate (and) stéarate de glycéryle | 1,00 |
| Alcool cétylique | 0,50 |
| Acétate de tocophéryle | 1,50 |
| Propylparaben / méthylparaben | 0,25 |
| Dioxyde de titane | 2,00 |
| Filtres solaires (anti UV-A et UVB) | 8,00 |
| Parfum | 0,30 |
| Eau q.s.p. | 100,00 |

## Revendications

1. Composition utilisable par voie topique pour le traitement d'affections dermatologiques, caractérisée en ce qu'elle comprend en combinaison au moins un alpha-hydroxy-acide, ou un de ses sels et esters acceptables, et au moins un céramide ou précurseur de céramide.

2. Composition selon la revendication 1, caractérisée en ce que l'alpha-hydroxy-acide est représenté par la formule générale (I) 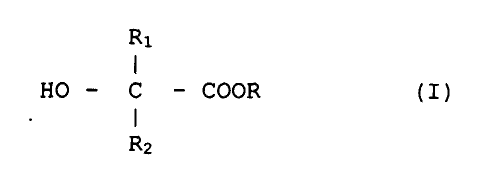 dans laquelle R₁ et R₂, identiques ou différents, représentent un atome d'hydrogène, un groupe alkyle linéaire ou ramifié comprenant 1 à 5 atomes de carbone éventuellement substitué par un groupe carboxyle, ou un groupe phényle, et R est un atome d'hydrogène, un groupe ammonium ou un métal alcalin ou alcalino-terreux.

3. Composition selon l'une quelconque des revendications précédentes, caractérisée en ce qu'elle comprend un alpha-hydroxy-acide, un sel d'ammonium d'alpha-hydroxy-acide, et un céramide ou un précurseur de céramide.

4. Composition selon l'une quelconque des revendications précédentes, caractérisée en ce qu'elle comprend un sel d'ammonium d'alpha-hydroxy-acide choisi parmi le lactate d'ammonium, le citrate d'ammonium, le glycolate d'ammonium, et le mandélate d'ammonium.

5. Composition selon l'une quelconque des revendications précédentes, caractérisée en ce qu'elle comprend un β-glucan ou un β-glucan carboxyméthylé.

6. Composition selon la revendication 1, caractérisée en ce que le céramide est un céramide 1, un céramide 3 ou un céramide 6.

7. Composition selon la revendication 1, caractérisée en ce que le précurseur de céramide est la sphingosine ou la phytosphingosine.

8. Composition selon l'une quelconque des revendications précédentes, caractérisée en ce qu'elle comprend entre 0,1 % et 12 % en poids d'alpha-hydroxy-acide et entre 0,01 % et 10 % en poids de céramide ou de précurseur de céramide, par rapport au poids total de la composition.

9. Composition selon la revendication 8, caractérisée en ce qu'elle comprend un ou plusieurs alpha-hydroxy-acides à raison de 0,1 % à 15 %, un sel d'ammonium d'alpha-hydroxy-acide à raison de 0,5 % à 50 % et un céramide à raison de 0,01 % à 5 % en poids.

10. Composition selon la revendication 8, caractérisée en ce qu'elle comprend entre 0,1 % et 6 % en poids d'alpha-hydroxy-acide, entre 0,5 % et 18 % en poids de sel d'ammonium d'alpha-hydroxy-acide, et entre 0,01 % et 1 % en poids de céramide, par rapport au poids total de la composition.

11. Composition selon l'une quelconque des revendications 1 à 7, caractérisée en ce qu'elle comprend entre 1 % et 18 % de sel d'ammonium d'alpha-hydroxy-acide, entre 0,01 % et 1 % de céramide, et entre 0,01 % et 1 % de β-glucan ou de β-glucan carboxyméthylé par rapport au poids total de la composition.

12. Composition selon l'une quelconque des revendications précédentes, caractérisée en ce qu'elle se présente sous forme de crème, lait, gel, lotion, masque ou pommade.
